# EUROPÄISCHE PATENTSCHRIFT

(11) **EP 1 795 226 B1**
(45) Veröffentlichungstag und Bekanntmachung des Hinweises auf die Patenterteilung: **18.04.2012**
(21) Anmeldenummer: 06025060.2
(22) Anmeldetag: 05.12.2006
(51) Int. Cl.: A61N 1/375

(54) **Gehäuse für medizinisches Implantat**
Housing for a medical implant
Boîte pour un implant médical

(30) Priorität: 08.12.2005 DE 102005058551; 24.01.2006 DE 102006003224
(43) Veröffentlichungstag der Anmeldung: 13.06.2007
(73) Patentinhaber: Biotronik CRM Patent AG, 6341 Baar (CH)
(72) Erfinder: Müller, Niels, 20357 Hamburg (DE); Schaldach, Max, Dr., 12359 Berlin (DE); Neumann, Wiebke, 12209 Berlin (DE); Uhrlandt, Werner, 12355 Berlin (DE); Starke, Marcel, 12167 Berlin (DE)
(74) Vertreter: Lindner-Vogt, Karin L.

(56) Entgegenhaltungen:
- EP-A2- 0 732 124
- WO-A-96/25978
- WO-A2-01/99239
- GB-A- 2 122 428
- US-A- 3 871 382
- US-A- 5 431 695
- US-A1- 2004 093 038
- US-B1- 6 505 072

## Beschreibung

Die Erfindung betrifft ein Gehäuse für ein medizinisches Implantat wie beispielsweise einen implantierbaren Herzschrittmacher, Defibrillator, Kardioverter oder dergleichen. Das Gehäuse umfasst ein Hohlgehäuse, welches beispielsweise der Aufnahme von Steuerelektronik, Kondensatoren und Batterien dient. Außerdem umfasst das Gehäuse einen Anschlusskörper, der an dem Hohlgehäuse befestigt ist, der wenigstens einen, in der Regel aber zwei bis vier in einer oder mehreren von außen zugänglichen Kavitäten des Anschlusskörpers angeordnete elektrischen Anschluss aufweist, der zum Anschließen einer oder mehrerer Elektrodenleitungen dient. Ein solcher Anschlusskörper wird auch als Header bezeichnet. Hohlgehäuse und Header ergeben zusammen ein hermetisch dichtes Gehäuse, welches im Inneren des Hohlgehäuses die genannten elektrischen Komponenten aufweist, die über Zuleitungen mit dem elektrischen Anschluss oder den elektrischen Anschlüssen in dem Header verbunden sind.

Das Hohlgehäuse ist in der Regel aus biokompatiblem Metall gefertigt. Die elektrische Verbindung der elektrischen Komponenten in dem Hohlgehäuse zu den im Header befindlichen elektrischen Zuleitungen zu den elektrischen Anschlüssen erfolgt in der Regel über Durchführungen, die in eine Wand des Hohlgehäuses eingelassen sind und einerseits das Hohlgehäuse hermetisch dicht abschließen und andererseits auch als Filterdurchführungen ausgebildet sein können, die eine elektrische Tiefpasswirkung haben.

Das Anschlussgehäuse beziehungsweise der Header besteht in der Regel aus transparenten, isolierendem Kunststoff. Die elektrischen Anschlüsse sind als Buchsen ausgeführt, die entsprechende Stecker von Elektrodenleitungen aufnehmen können. Aufgrund der Transparenz des Anschlussgehäuses ist von außen sichtbar, ob ein Elektrodenleitungsstecker weit genug in die Buchse eines jeweiligen elektrischen Anschlusses eingeführt ist.

An die Qualität eines Gehäuses für ein medizinisches Implantat werden hohe Anforderungen gestellt. Insbesondere müssen Hohlgehäuse und Anschlusskörper über Jahre hinweg zuverlässig und dicht zusammenwirken. Der Header selbst muss über lange Zeit stabil und passgenau sein.

Um die vorgenannten Anforderungen zu erfüllen, sind aus dem Stand der Technik verschiedene Ansätze bekannt. Zum einen ist es bekannt, das Anschlussgehäuse an das Hohlgehäuse direkt anzugießen. Dazu wird das Hohlgehäuse mit daran befestigten Zuleitungen für die elektrischen Anschlüsse und Kontaktbuchsen für die Elektrodenleitungsstecker in eine Gussform eingesetzt und ein Basiskörper des Anschlussgehäuses in einem Stück durch Füllen der geschlossenen Gussform mit flüssigen Kunststoff erzeugt. Der flüssige Kunststoff wird in der Gussform aushärten gelassen und ergibt im Ergebnis - nach Entfernen der Gussform - einen in einem Arbeitsschritt hergestellten, unmittelbar fest mit dem Hohlgehäuse verbundenen Anschlusskörper.

Alternativ sind vormontierte Header bekannt, bei denen die elektrischen Bestandteile des Headers wie beispielsweise elektrische Kontaktbuchsen oder elektrische Zuleitungen zunächst in vorgefertigte Spritzgussteile aus Kunststoff eingesetzt werden. Solche mehrteiligen, vormontierbaren Header sind beispielsweise aus WO 01/99239, EP 0 429 024, US 5,282,841 oder US 6,205,358 bekannt.

Aufgabe der Erfindung ist es, ein Gehäuse für ein medizinisches Implantat wie einen Herzschrittmacher oder dergleichen und ein Verfahren zu dessen Herstellung zu schaffen, welches eine möglichst einfache, wenig fehlerträchtige und kostengünstige Herstellung erlaubt und zu einem Gehäuse mit den geforderten Eigenschaften führt.

Erfindungsgemäß wird diese Aufgabe durch ein Gehäuse der eingangs genannten Art erreicht, bei dem die elektrischen Zuleitungen mit den elektrischen Kontakten verschweißt und die miteinander verschweißten elektrischen Zuleitungen und elektrischen Kontakte in den Basiskörper eingegossen oder umspritzt und so in ihrer endgültigen Position durch zwei- oder mehrstufiges Umspritzen oder Umgießen fixiert sind. Der Basiskörper ist mit dem Hohlgehäuse mittels eines Klebstoffes zwischen dem Hohlgehäuse und dem Anschlusskörper ' verklebt.

Auf diese Weise entsteht ein Gehäuse für einen Herzschrittmacher, bei dem der Anschlusskörper vorgefertigt ist, bevor er mit dem Hohlgehäuse durch Kleben verbunden wird. Dies erlaubt es, den Anschlusskörper in jeder Hinsicht zu testen, bevor er durch Verbinden mit dem Hohlgehäuse weiter verarbeitet wird. Dabei ist zu berücksichtigen, dass das Hohlgehäuse bereits die gesamten, kostspieligen elektrischen Bestandteile aufweisen muss, bevor das Hohlgehäuse mit dem Anschlusskörper (Header) verbunden werden kann. Daher ist es vorteilhaft, wenn der Anschlusskörper hinsichtlich all seiner Eigenschaften getestet werden kann bevor er mit dem Hohlgehäuse durch Kleben verbunden wird. Durch die Klebeverbindung zwischen Anschlusskörper und Hohlgehäuse wird gleichzeitig die mechanische Verbindung zwischen Anschlusskörper und Hohlgehäuse hergestellt und außerdem die geforderte Dichtigkeit zwischen Anschlusskörper und Hohlgehäuse sichergestellt. Der Klebstoff zwischen Anschlusskörper und Hohlgehäuse wirkt somit gleichermaßen als Mittel zur Übertragung mechanischer Kräfte vom Anschlusskörper zum Hohlgehäuse und als Dichtmittel.

Der Basiskörper ist wenigstens teilweise transparent und erlaubt einen Blick von außerhalb des Anschlusskörpers auf den elektrischen Anschluss. Vorzugsweise besteht der Basiskörper aus einem transparenten Kunststoff, der als Gießharz oder als Thermoplast verarbeitbar ist.

Das erfindungsgemäße Verfahren besteht aus den aufeinander folgenden Schritten: Die elektrischen Zuleitungen, elektrischen Kontakte und die Antenne werden in einer Spritzgussform fixiert, in einem 1. Spritzvorgang werden die elektrischen Zuleitungen, die elektrischen Kontakte und die Antenne teilweise umspritzt oder umgossen, anschließend wird der Spritzling in der Spritzgussform fixiert und danach werden in einem 2. Spritzvorgang die elektrischen Zuleitungen, die elektrischen Kontakte und die Antenne vollständig umspritzt oder umgossen, um so die endgültige Form des Anschlusskörpers zu erhalten.

Zusätzlich können vorzugsweise vor der Fixation der elektrischen Zuleitungen, elektrischen Kontakte und der Antenne, die Zuleitungen und die Kontakte durch eine Fügetechnik - besonders bevorzugt durch Schweißen - fixiert werden.

Die Fixierung der elektrischen Zuleitungen, elektrischen Kontakte und der Antenne kann durch Haltemittel erfolgen. Die Haltemittel werden dabei so positioniert, dass die Enden der elektrischen Zuleitungen und der Antenne fixiert werden. Dadurch entsteht nach dem Entfernen des Haltemittels eine nicht umspritzter Hohlraum, in dem dann beispielsweise durch Schweißen ein elektrischer Kontakt mit Kontaktstiften hergestellt werden kann. Diese Kontaktstifte ermöglichen den elektrischen Kontakt aus dem Hohlgehäuse heraus. Dieser Hohlraum kann vorzugsweise in einem nachgelagerten weiteren Spritzvorgang ausgefüllt werden.

In einer weiteren bevorzugten Ausgestaltung des Verfahrens werden einer oder mehrere der elektrischen Kontakte mit einer oder mehreren Klemmschrauben, welche zur Schraubkontaktierung einer Elektrodenleitung in den Steckbuchsen dienen, vormontiert und anschließend in einem weiteren Spritzgussvorgang ein Deckel zur Abdichtung der Schraubkontaktierung nach außen an- oder umgespritzt.

Vorzugsweise wird für den 2. Spritzvorgang ein anderer Kunststoff verwendet als für den 1. Spritzvorgang. Damit können die unterschiedlichen Anforderungen bei der inneren Fixierung und Isolation sowie beim äußeren Schutz des Basiskörpers besser erfüllt werden.

Das soeben genannte Verfahren kann auch vorteilhaft in einem Spritzvorgang ausgeführt werden, indem die Zuleitungen mit den elektrischen Kontakten verschweißt werden, die Zuleitungen und die Antennen zueinander fixiert werden und dann in einem Spritzvorgang umgossen oder umspritzt werden.

Der Klebstoff, mit dem der Anschlusskörper mit dem Hohlgehäuse verklebt ist, ist vorzugsweise Epoxidharz oder enthält Epoxidharz. Epoxidharz zeichnet sich durch günstige Verarbeitungseigenschaften wie auch durch günstige Eigenschaften hinsichtlich der erzielbaren Festigkeit und hinsichtlich der Biokompatibilität aus. In Bezug auf die erzielbare Festigkeit ist es besonders vorteilhaft, dass Epoxidharz sowohl sehr gut an verschiedenen Oberflächen haftet, also große Adhäsionskräfte erlaubt, als auch selbst eine gute innere Zugfestigkeit besitzt, also große Kohäsionskräfte entwickelt.

Besonders bevorzugt ist eine lichthärtende Variante von Epoxidharz oder ein anderer Klebstoff, der bei Bestrahlung mit elektromagnetischer Strahlung in einem ultravioletten Wellenlängenbereich aushärtet. UV-lichthärtende Klebstoffe lassen sich besonders günstig und zuverlässig verarbeiten.

Im Zusammenhang mit dem Einsatz UV-lichthärtender Klebstoffe ist es vorteilhaft, wenn der Basiskörper des Anschlusskörpers aus einem Material besteht, welches in demjenigen Wellenlängenbereich, in dem die für das Aushärten des Klebstoffes erforderliche Beleuchtung liegt, eine Transmission aufweist, die ausreichend hoch ist, um ein Aushärten des Klebstoffes bei einer gegebenen Beleuchtungsintensität in höchstens der vierfachen der für diese Beleuchtungsintensität angegebenen Zeitdauer zu erlauben. Auf diese Weise wird sichergestellt, dass der Klebstoff in ausreichend kurzer Zeit zuverlässig über die gesamte Fläche der Klebverbindung zwischen Hohlgehäuse und Anschlusskörper aushärtet.

Darüber hinaus ist vorteilhaft, wenn der Basiskörper wenigstens teilweise transparent ist und einen Blick von außerhalb des Anschlusskörpers auf den elektrischen Anschluss oder die elektrischen Anschlüsse erlaubt.

Ein geeignetes Kunststoffmaterial für einen Basiskörper aus transparentem Kunststoff ist Polyurethan oder Epoxidharz. Ein ebenso geeignetes, alternatives Kunststoffmaterial für einen Basiskörper ist auch Polykarbonat oder Polyurethan.

Neben denjenigen Kavitäten, die als Steckbuchsen mit darin angeordnetem elektrischen Kontakt, insbesondere in Form von Kontaktbuchsen, zur Aufnahme eines jeweiligen Anschlusssteckers einer Elektrodenleitung dienen, weist der Anschlusskörper vorzugsweise wenigstens eine weitere - nach außen offene - Kavität auf, die der Aufnahme von Funktionsbaugruppen, beispielsweise in Form eines Röntgenmarkers oder eines Deckels mit mindestens einem Dichtstopfen, dient. Diese vormontierten Funktionsbaugruppen werden nach Gießen des Basiskörpers in die entsprechenden Kavitäten eingesetzt oder eingeschoben und in einer besonderen Ausprägung durch eine Passung (Übergang- oder Presspassung), einen "Snap In" oder auch einen "Press Fit" in Position gehalten, bis sie durch die Zugabe von Klebstoff / Dichtmittel dauerhaft und dicht im oder am Basiskörper fixiert werden. Auf diese Weise entsteht ein Anschlusskörper, der auch im Bereich der Kavitäten nach außen abgedichtet ist.

Die Kavitäten zur Aufnahme von vormontierten Funktionsbaugruppen - vorzugsweise die des Röntgenmarkers - und der Hohlraum, der durch die Entfernung des Haltemittels entsteht, werden vor der Abdichtung vorzugsweise für ein Verfahren zur Verklebung des Anschlusskörpers mit dem Hohlgehäuse genutzt, indem einer der vorgenannten Kleber über diese Kavitäten und/oder den Hohlraum appliziert wird und durch Kapillarwirkung in den Klebespalt zwischen dem Anschlussgehäuse und dem Hohlgehäuse gelangt.

Wie bereits erläutert, ist es vorteilhaft, wenn ein jeweiliger elektrischer Anschluss in dem Anschlusskörper von wenigstens einer Steckbuchse gebildet ist, die von einer Kavität im Basiskörper gebildet ist und einen in der Steckbuchse angeordneten elektrischen Kontakt aufweist. Der elektrische Kontakt ist vorzugsweise eine Kontaktbuchse. Die Steckbuchse besitzt eine Form, die zur Aufnahme eines Anschlusssteckers einer Elektrodenleitung geeignet ist.

Die elektrischen Kontakte der Steckbuchsen sind jeweils vorzugsweise mit elektrischen Zuleitungen verbunden, die von flachen Leitungen aus leitfähigem und biokompatiblem Material, bevorzugt Metall, gebildet sind. Bekannte Materialien für die elektrischen Zuleitungen sind korrosionsgehemmte Stähle (z.B. 316 L), Kobalt-Basislegierungen (z.B. MP35N), Reintitan (Grade 1-4), Titanlegierungen (Grade 5 TiAI6V4), Reinniob, Nioblegierungen oder andere als biokompatibel bekannte Metalle. Die flachen Leitungen sind vorzugsweise jeweils mit einer Kontaktbuchse einer jeweiligen Steckbuchse verschweißt.

Vorzugsweise ist neben den elektrischen Zuleitungen eine weitere, vorzugsweise flache Leitung aus leitfähigem biokompatiblem Material, bevorzugt Metall vorgesehen, die als Antenne für einen Telemetriesender und gegebenenfalls -empfänger im Inneren des Hohlgehäuses dient. Diese Antenne wird zusammen mit den Zuleitungen im Basiskörper eingegossen und so in ihrer endgültigen Position fixiert. Bekannte Materialien für die Antenne sind korrosionsgehemmte Stähle (z.B. 316 L), Kobalt-Basislegierungen (z.B. MP35N), Reintitan (Grade 1-4), Titanlegierungen (Grade 5 TiA16V4), Reinniob, Nioblegierungen oder andere als biokompatibel bekannte Metalle. Die flachen Leitungen sind vorzugsweise jeweils mit einer Kontaktbuchse einer jeweiligen Steckbuchse verschweißt.

Vorzugsweise liegen die elektrischen Zuleitungen und die Antenne aus flachen Leitungen aus leitfähigem Material in einer Ebene und sind vorzugsweise im Nutzen hergestellt. Als Nutzen wird die Ein- oder Mehrfachfertigung von Bauteilen aus einem strangförmigen Material - zum Beispiel Bänder aus einem der genannten Materialien - verstanden. Dabei ist eine möglichst hohe Minimalisierung der Arbeitsschritte möglich.

Um eine zuverlässige und passgenaue Montage des vorgefertigten Anschlusskörpers am Hohlgehäuse sicherzustellen, besitzt der Basiskörper auf seiner dem Hohlgehäuse zugewandten Seite vorzugsweise zwei Kavitäten, die der Aufnahme von an dem Hohlgehäuse befestigten Ankern in Form von vom Hohlgehäuse abstehenden Stiften dienen, die zusammen mit den Kavitäten eine genaue Positionierung des Basiskörpers insbesondere auch während des Verklebens des Anschlusskörpers mit dem Hohlgehäuse bewirken und die Übertragung von Biegekräften zwischen Hohlgehäuse und Anschlusskörper verbessern. Die Vorfixierung des Basiskörpers mit den Ankern kann durch eine Passung (Übergang- oder Presspassung), durch "Press Fit" oder durch "Snap In" erfolgen.

In einer vorteilhaften Ausführungsvariante besitzt der Anschlusskörper eine weitere Kavität zur Aufnahme eines Röntgenmarkers. Diese Kavität ist vorzugsweise mit Kleber, einem Dichtstoff oder einem Deckel dicht zu verschließen und erlaubt es, Röntgenmarker zur individuellen Kennzeichnung eines Gehäuses nach Herstellen des Basiskörpers einzusetzen.

Hinsichtlich Form und Volumen ist das Gehäuse vorzugsweise ein Gehäuse für einen Herzschrittmacher, einen Kardioverter/Defibrillator oder eine Kombination aus beiden. In diesem Sinne wird auch ein Herzschrittmacher oder Kardioverter/Defibrillator mit einem hier beanspruchten Gehäuse beanspruchte.

Die Erfindung soll nun anhand eines Ausführungsbeispiels mit Bezug auf die Fig.en näher erläutert werden. Von den Fig.en zeigen:
- Fig. 1:: ein Gehäuse eines Herzschrittmachers mit einem Hohlgehäuse aus biokompatiblem Metall und mit einem Anschlusskörper beziehungsweise Header zum Anschließen von Elektrodenleitungen;
- Fig. 2:: das Gehäuse aus Fig. 1, dargestellt in einer Explosionszeichnung mit voneinander getrenntem Hohlgehäuse und Anschlusskörper;
- Fig. 3:: das Gehäuse aus Fig. 1 mit einer Darstellung des Anschlusskörpers in halbtransparenter Darstellungsweise;
- Fig. 4:: eine Darstellung von elektrischen Zuleitungen, elektrischen Kontakten und Antenne im vormontierten Zustand vor dem Gießen oder Umspritzen und Herstellen des Basiskörpers;
- Fig. 5:: den Basiskörpers nach dem 1. Spritzvorgang;
- Fig. 6:: den Anschlusskörpers nach dem 2. Spritzvorgang; und
- Fig. 7:: eine Darstellung der dem Hohlgehäuse zugewandten Seite des Anschlusskörpers

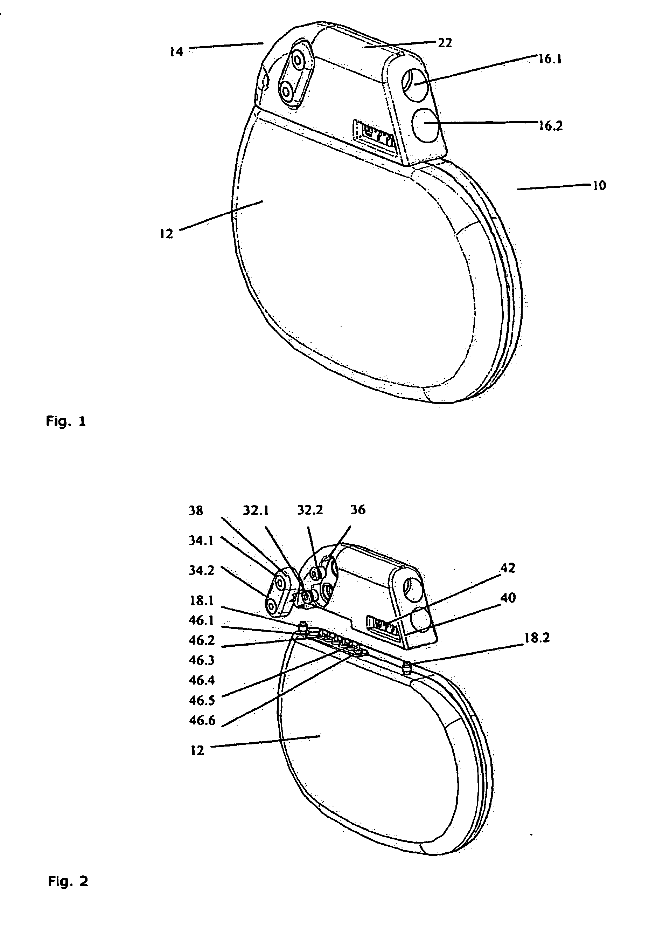

Fig. 1 zeigt ein medizinisches Implantat mit einem Gehäuse 10, welches ein Hohlgehäuse 12 zur Aufnahme von Batterien, Kondensatoren und Steuerelektronik aufweist sowie einen Anschlusskörper 14 - auch Header genannt - der zwei Steckbuchsen 16.1 und 16.2 aufweist, in denen Stecker von Elektrodenleitungen einzustecken sind.

Erfindungsgemäß ist der Anschlusskörper 14 mit dem Hohlgehäuse 12 verklebt. Dazu ist eine Klebeschicht aus beispielsweise Epoxidharz vorgesehen, die in Fig. 1 nicht erkennbar ist und die sich zwischen den einander gegenüberliegenden Flächen des Anschlussgehäuses 14 und des Hohlgehäuses 12 befindet.

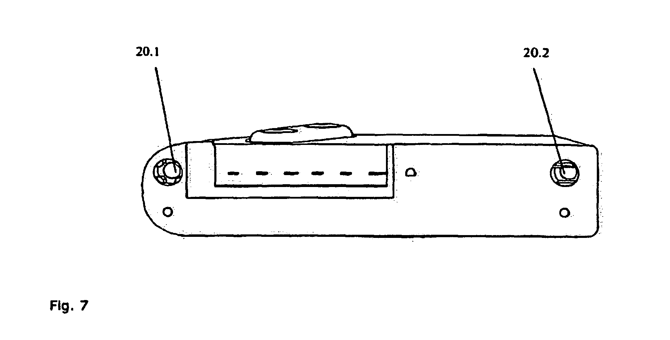

Um das Verkleben von Anschlusskörper 14 und Hohlgehäuse 12 zu erleichtern, sind - wie in Fig. 2 zu erkennen ist - am Hohlgehäuse 12 zwei Anker 18.1 und 18.2 vorgesehen, die in entsprechende Kavitäten eines Basiskörpers 22 des Anschlussgehäuses 14 eingreifen. Diese Kavitäten 20.1 und 20.2 sind in Fig. 7 dargestellt. Außerdem sind in Fig. 2 Klemmschrauben 32.1 und 32.2 in einer Kavität 36 dargestellt, die mit einem Deckel 38 und darin integrierten Dichtstopfen 34.1 und 34.2 verschließbar sind. In einer weiteren Kavität 40 des Anschlusskörpers 14 ist ein Röntgenmarker 42 angeordnet. Das Hohlgehäuse 12 besteht aus zwei Hohlgehäusehälften und enthält eine Durchführung, mit der elektrische Anschlüsse aus dem Inneren des Hohlgehäuses 12 zu sechs in einer Reihe angeordneten elektrischen Kontaktstiften 46.1 bis 46.6 geführt werden.

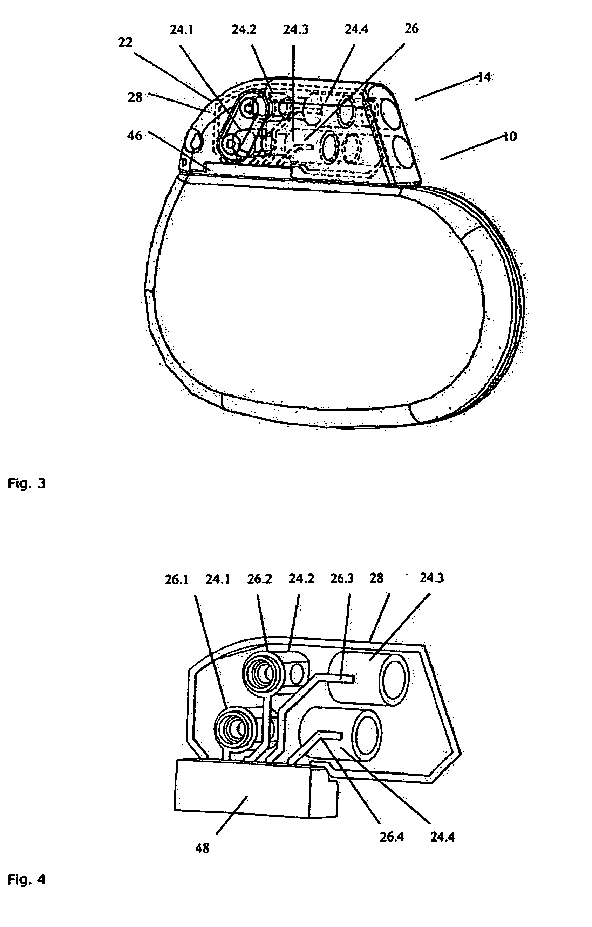

Wie Fig. 3 und 4 zu entnehmen ist, wird der Anschlusskörper 14 von einem transparenten Basiskörper 22 gebildet, in dem die Kontaktbuchsen 24.1 bis 24.4 sowie elektrischen Zuleitungen 26.1 bis 26.4 und einer Antenne 28 eingegossen oder umspritzt sind. Die Antenne 28 wird mit den Kontaktstiften 46.1 und 46.6 und die elektrischen Zuleitungen 26.1 bis 26.4 mit den Kontaktstiften 46.2 bis 46.4 kontaktiert. Die elektrischen Zuleitungen 26.1 bis 26.4 führen an den entgegengesetzten Enden zu den Kontaktbuchsen 24.1 bis 24.4.

Die Kontaktstifte 46.1 bis 46.6 werden nach dem Verkleben des Anschlusskörpers 14 mit dem Hohlgehäuse 12 durch Verschweißen mit den elektrischen Zuleitungen 26.1 bis 26.4 sowie der Antenne 28 verbunden. Dazu ist auf der dem Hohlgehäuse 12 zugewandten Seite des Anschlusskörpers 14 eine zunächst offene Kavität 46 vorgesehen, die nach dem Verschweißen der Kontaktstifte 46.1 bis 46.6 mit den Zuleitungen 26.1 bis 26.4 und der Antenne 28 vergossen wird.

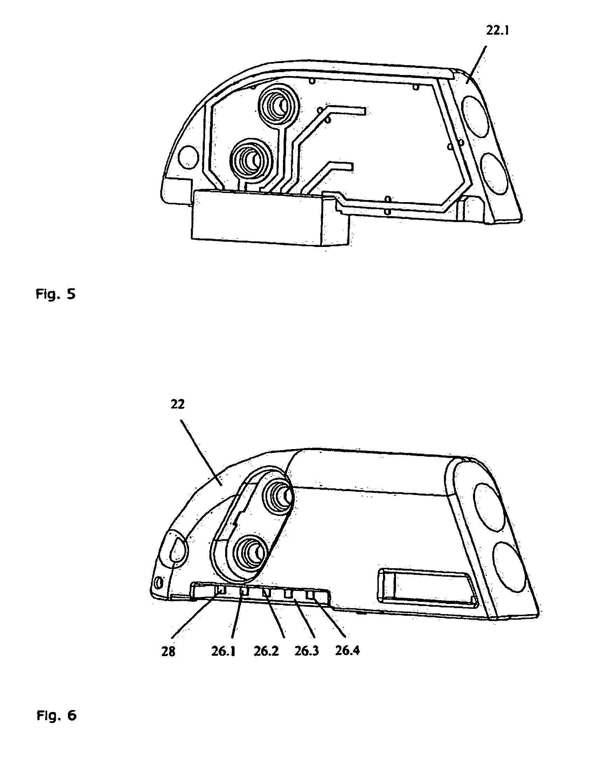

Um den Anschlusskörper 14 herzustellen, werden zunächst die elektrischen Zuleitungen 26.1 bis 26.4 und die Antenne 28 hergestellt, die über eine Halterung oder gemeinsame Anbindung 48 miteinander verbunden sind. Die elektrischen Zuleitungen 26.1 bis 26.4 werden mit den Kontaktbuchsen 24.1 bis 24.4 verschweißt. Anschließend wird Anbindung 48 zusammen mit der Antenne 28 und den elektrischen Zuleitungen 26.1 bis 26.4 in eine Gussform eingesetzt und dort festgehalten. Mit Hilfe dieser Gussform wird zunächst eine erste Hälfte 22.1 des Basiskörpers 22 gegossen. Dies erlaubt es, die nur wenige zehntel Millimeter dicken elektrischen Zuleitungen 26.1 bis 26.4 sowie die Antenne 28 während des Vergießens mit Hilfe von Stiften in Position zu halten. Nachdem die erste Gusshälfte 22.1 des Basiskörpers 22 ausreichend ausgehärtet ist, wird die zweite Hälfte des Basiskörpers 22 an die erste Gusshälfte 22.1 angegossen, so dass der fertige Basiskörper 22 entsteht, siehe Fig. 6. In den Basiskörper 22 sind die elektrischen Zuleitungen 26.1 bis 26.4, die Antenne 28 sowie die Kontaktbuchsen 24.1 bis 24.4 fest eingegossen. Nachdem auf diese Weise der Basiskörper 22 vollständig hergestellt und ausgehärtet ist, können die durch den Basiskörper 22 fixierten elektrischen Zuleitungen 26.1 bis 26.4 und die Antenne 28 von der Halterung 48 abgetrennt werden.

Anschließend kann der vorgefertigte Anschlusskörper 14 auf das Hohlgehäuse 12 aufgesetzt werden. Die Antenne 28 und die elektrischen Zuleitungen 26.1 und 26.4 werden mit den Kontaktstiften 46.1 bis 46.6 verschweißt. Nach Montage der Klemmschrauben 32.1 und 32.2 wird die Kavität 36 mit dem Deckel 38, welcher zwei Dichtstopfen 34.1 und 34.2 enthält, verschlossen.
Um den so entstandenen Herzschrittmacher kennzeichnen zu können, kann schließlich ein individueller Röntgenmarker 42 in die Kavität 40 eingesetzt werden. Nach dem Verschweißen der Kontaktstifte mit den elektrischen Zuleitungen 26.1 bis 26.4 und der Antenne 28 wird die Kavität 48 vergossen oder verklebt. Das Anschließen der Elektrodenleitungen an den Herzschrittmacher wird durch Einstecken von entsprechenden Elektrodenleitungssteckern in die Steckbuchsen 16.1 und 16.2 und die Fixierung der Elektrodenleitungsstecker mit Hilfe der Klemmschrauben 32.1 und 32.2 bewerkstelligt.

In einem anschließenden Prozess wird der Anschlusskörper 14 mit dem Hohlgehäuse 12 verklebt, wobei auch die Kavitäten 46 und 40 mit dem Kleber verfüllt werden. Auch der Deckel 38 kann in diesem Zusammenhang mit dem Anschlusskörper verklebt werden.

Ein geeignetes Material für den Basiskörper 22 ist transparentes Polyurethan, welches leicht vergossen werden kann und die gewünschten mechanischen und elektrischen sowie optischen Eigenschaften aufweist, nämlich transparent ist. Ein besonders geeignetes Polyurethan ist ein aliphatisches oder aromatisches Acryl-Polyester Polyurethan.

Wie bereits erwähnt, ist ein geeigneter Klebstoff zum Verkleben des Anschlusskörpers 14 mit dem Hohlgehäuse 12 Epoxidharz. Besonders bevorzugt ist ein lichthärtendes Epoxidharz, welches bei Beleuchtung beziehungsweise Bestrahlung mit ultravioletter Strahlung aktiviert wird und aushärtet.

## Patentansprüche

1. Gehäuse (10) für ein medizinisches Implantat, wie einen Herzschrittmacher, Defibrillator, Kardioverter oder dergleichen, wobei
- das Gehäuse ein Hohlgehäuse (12) und einen an dem Hohlgehäuse (12) befestigten Anschlusskörper (14) aufweist, der wenigstens einen in einer von außen zugänglichen Kavität des Anschlusskörpers angeordneten elektrischen Anschluss zum Anschließen einer Elektrodenleitung besitzt, und
- der Anschlusskörper (14) einen Basiskörper (22) aus elektrisch isolierendem Kunststoff umfasst, der mit dem Hohlgehäuse (12) verbunden ist und elektrische Zuleitungen (26) sowie mit den elektrischen zuleitungen risch leitend verschweißte elektrische Kontakte (24) für den elektrischen Anschluss derart tragt , dass die Kontakte (24) über die elektrischen Zuleitungen (26) mit elektrischen Komponenten im Inneren des Hohlgehäuses (12) zu verbinden sind, wobei die miteinander verschweißten elektrischen zuleitungen und elektrischen kontakte in den Basiskörper eingegosen oder umspritzt und so in ihrer endgültigen Position fixiert sind, **dadurch gekennzeichnet, dass**
die elektrischen Zuleitungen (26) und die elektrischen Kontakte (24) durch zwei- oder mehrstufiges Umspritzen oder Umgießen fixiert send.

2. Gehäuse nach Anspruch 1, **dadurch gekennzeichnet, dass** der Basiskörper (22) wenigstens teilweise transparent ist und einen Blick von außerhalb des Anschlusskörpers auf den elektrischen Anschluss erlaubt.

3. Gehäuse nach einem der Ansprüche 1 oder 2, **dadurch gekennzeichnet, dass** der Basiskörper (22) aus einem transparenten Kunststoff besteht, der als Gießharz oder als Thermoplast verarbeitbar ist.

4. Gehäuse nach Anspruch 3, **dadurch gekennzeichnet, dass** der Basiskörper (22) aus einem transparenten Kunststoff besteht, der Polyurethan und / oder Epoxidharz enthält.

5. Gehäuse nach einem der Ansprüche 1 bis 4, **dadurch gekennzeichnet, dass** der Anschlußkörper (14) mit dem Hohlgehäuse (12) mittels eines Klebstoffes verklebt ist.

6. Gehäuse nach einem der Ansprüche 1 bis 5, **dadurch gekennzeichnet, dass** der Basiskörper mindestens eine weitere, nach außen ursprünglich offene Kavität (36, 40) zur Aufnahme von Funktionsbaugruppen (34) aufweist.

7. Gehäuse nach Anspruch 6, **dadurch gekennzeichnet, dass** die Funktionsbaugruppe ein Röntgenmarker (42) ist.

8. Gehäuse nach Anspruch 6, **dadurch gekennzeichnet, dass** die Funktionsbaugruppe ein Deckel (38) mit mindestens einem Dichtstopfen (34.1, 34.2) ist.

9. Gehäuse nach Anspruch 8, **dadurch gekennzeichnet, dass** der Deckel (38) mit dem Basiskörper (22) mittels eines Klebstoffes dicht verklebt ist.

10. Gehäuse nach einem der Ansprüche 1 bis 9, **dadurch gekennzeichnet, dass** der elektrische Anschluss von wenigstens einer Steckbuchse (16) und einem in der Steckbuchse (16) angeordneten elektrischen Kontakt (24) gebildet ist, wobei die Steckbuchse (16) zur Aufnahme eines Anschlusssteckers einer Elektrodenleitung ausgebildet ist.

11. Gehäuse nach einem der Ansprüche 1 bis 10, **dadurch gekennzeichnet, dass** die elektrischen Zuleitungen (26) in dem Anschlusskörper (14) von flachen leitungen aus leitfähigem Material gebildet sind, vorzugsweise umfassend:
- Korrosionsgehemmte Stähle,
- Reintitan,
- Eine Titanlegierung,
- Reinniob,
- Eine Nioblegierung oder
- Eine eisenfreie Kobalt-Basis-Legierung.

12. Gehäuse nach einem der Ansprüche 1 bis 11, **dadurch gekennzeichnet, dass** zusammen mit den Zuleitungen eine eine Antenne (28) bildende Leitung in den Basisköper (22) eingegossen oder zusammen mit diesem umspritzt und so in ihrer endgültigen Position fixiert ist.

13. Gehäuse nach Anspruch 12, **dadurch gekennzeichnet, dass** die Antenne (28) von einer flachen Leitung aus leitfähigem Material gebildet ist, vorzugsweise umfassend:
- Korrosionsgehemmte Stähle,
- Reintitan,
- Eine Titanlegierung,
- Reinniob,
- Eine Nioblegierung oder
- Eine eisenfreie Kobalt-Basis-Legierung.

14. Gehäuse nach einem der Ansprüche 11 bis 13, **dadurch gekennzeichnet, dass** die elektrischen Zuleitungen (26) und / oder die Antenne (28) aus flachen Leitungen aus leitfähigem Material in einer Ebene liegen und vorzugsweise im Nutzen hergestellt sind.

15. Gehäuse nach einem der Ansprüche 1 bis 14, **dadurch gekennzeichnet, dass** der Basiskörper (22) auf seiner dem Hohlgehäuse (12) zugewandten Seite zwei Kavitäten (20) zur Aufnahme von an dem Hohlgehäuse (12) befestigten Ankern (18) aufweist, die zusammen mit den Kavitäten (20) eine genaue Positionierung des Basiskörpers (22) relativ zum Hohlgehäuse (12) vor und während des Verklebens des Anschlusskörpers (14) mit dem Hohlgehäuse (12) bewirken.

16. Gehäuse nach einem der Ansprüche 1 bis 15, **dadurch gekennzeichnet, dass** das Gehäuse (10) das Gehäuse eines implantierbaren Herzschrittmachers oder eines implantierbaren Kardioverters/Defibrillators oder einer Kombination aus beidem ist.

17. Verfahren zur Herstellung eines Gehäuses für ein medizinisches Implantat nach einem der Ansprüche 1 bis 16 mit einem Hohlgehäuse (12) und einem daran befestigten Anschlusskörper (14), der einen Basiskörper (22) aus einem elektrisch isolierenden Kunststoff umfasst, mit den folgenden Schritten:
- Fixieren von elektrischen Zuleitungen (26), elektrischen Kontakten (24) und Antenne (28) in einer Spritzgussform,
- 1. Spritzvorgang, bei dem die elektrischen Kontakte (24), die elektrischen Zuleitungen (26) und die Antenne (28) teilweise umspritzt oder umgossen werden,
- Fixierung des Spritzlings in der Spritzgussform,
- 2. Spritzvorgang, bei dem die elektrischen Zuleitungen (26), die elektrischen Kontakte (24) und die Antenne (28) vollständig umgossen werden, um die endgültige Form des Anschlusskörpers zu erhalten.

18. Verfahren nach Anspruch 17, **gekennzeichnet durch** folgenden vorgelagerten Schritt:
- Fügen der elektrischen Kontakte (24) mit den elektrischen Zuleitungen (26), vorzugsweise **durch** Schweißen.

19. Verfahren nach Anspruch 17, **dadurch gekennzeichnet, dass** die elektrischen Zuleitungen (26), die elektrischen Kontakten (24) und die Antenne (28) mit Haltemitteln (48) in der Spritzgußform fixiert werden.

20. Verfahren nach Anspruch 19, **gekennzeichnet durch** folgende nachgelagerte Schritte:
- Entfernen der Haltemittel (48), und
- weiterer Spritzvorgang zum Ausfüllen der beim Entfernen der Haltemittel (48) entstandenen Hohlräume (46).

21. Verfahren nach Anspruch 17 **gekennzeichnet durch** folgende weitere Schritte:
- Vormontage einer oder mehrerer der elektrischen Kontakte (24.1, 24.2) mit einer oder mehreren Klemmschrauben (32.1, 32.2),
- Spritzgussvorgang zum An- oder Umspritzen des Deckels (38) und zur Abdichtung einer Schraubkontaktierung nach rußen.

22. Verfahren nach Anspruch 17, **dadurch gekennzeichnet, dass** für den 2. Spritzvorgang ein anderer Kunststoff verwendet wird als für den 1. Spritzvorgang.

## Claims

1. A housing (10) for a medical implant, such as a cardiac pacemaker, defibrillator, cardioverter or the like, wherein
- the housing comprises a hollow housing (12) and a connecting element (14) attached to the hollow housing (12), which has at least one electrical connection disposed in a cavity of the connecting element, which is accessible from the outside, for connection to an electrode lead, and
- the connecting element (14) has a main body (22) made of electrically insulating plastic, which is connected to the hollow housing (12) and carries electrical leads (26) and, welded to the electrical leads in an electrically conductive manner, electrical contacts (24) for the electrical connection in such a manner that the contacts (24) can be connected by way of the electrical supply leads (26) to electrical components in the interior of the hollow housing (12), wherein the electrical supply leads, which are welded to one another, and electrical contacts are cast in the main body or are encased by way of injection molding and are thereby fixed in the final position thereof, **characterized in that** the electrical supply leads (26) and the electrical contacts (24) are fixed by way of two-staged or multiple-staged encasing by way of injection molding or casting.

2. The housing according to claim 1, **characterized in that** the main body (22) is at least partially transparent and permits a view of the electrical connection from outside of the connecting element.

3. The housing according to one of the claims 1 or 2, **characterized in that** the main body (22) is made of a transparent plastic which can be processed as casting resin or a thermoplastic material.

4. The housing according to claim 3, **characterized in that** the main body (22) is made of a transparent plastic containing polyurethane and/or epoxy resin.

5. The housing according to one of the claims 1 to 4, **characterized in that** the connecting element (14) is bonded to the hollow housing (12) by way of an adhesive.

6. The housing according to one of the claims 1 to 5, **characterized in that** the main body comprises at least one further cavity (36, 40), which is originally open toward the outside, for accommodating functional assemblies (34).

7. The housing according to claim 6, **characterized in that** the functional assembly is an x-ray marker (42).

8. The housing according to claim 6, **characterized in that** the functional assembly is a cover (38) comprising at least one sealing stopper (34.1, 34.2).

9. The housing according to claim 8, **characterized in that** the cover (38) is bonded in a sealed manner to the main body (22) by way of an adhesive.

10. The housing according to one of the claims 1 to 9, **characterized in that** the electrical connection is formed by at least one socket (16) and an electrical contact (24) disposed in the socket (16), wherein the socket (16) is designed to accommodate a connecting plug of an electrode lead.

11. The housing according to one of the claims 1 to 10, **characterized in that** the electrical supply leads (26) in the connecting element (14) are formed of flat leads made of conductive material, preferably comprising:
- corrosion-inhibited steels,
- pure titanium,
- a titanium alloy,
- pure niobium,
- a niobium alloy or
- a nonferrous cobalt master alloy.

12. The housing according to one of the claims 1 to 11, **characterized in that**, together with the supply leads, a lead which forms an antenna (28) is cast into the main body (22) or is encased together therewith by way of injection molding and is thereby fixed in the final position thereof.

13. The housing according to claim 12, **characterized in that** the antenna (29) is formed by a flat lead made of conductive material, preferably comprising:
- corrosion-inhibited steels,
- pure titanium,
- a titanium alloy,
- pure niobium,
- a niobium alloy or
- a nonferrous cobalt master alloy.

14. The housing according to one of the claims 11 to 13, **characterized in that** the electrical supply leads (26) and/or the antenna (28) comprising flat leads made of conductive material lie in a plane and are preferably produced in cutouts.

15. The housing according to one of the claims 1 to 14, **characterized in that** the main body (22) comprises, on the side thereof facing the hollow housing (12), two cavities (20) for accommodating armatures (18) fastened to the hollow housing (12), which, together with the cavities (20), bring about an exact positioning of the main body (22) relative to the hollow housing (12) before and during the bonding of the connecting element (14) to the hollow housing (12).

16. The housing according to one of the claims 1 to 15, **characterized in that** the housing (10) is the housing of an implantable cardiac pacemaker or an implantable cardioverter/defibrillator or a combination of the two.

17. A method for the production of a housing for a medical implant according to one of the claims 1 to 16, comprising a hollow housing (12) and a connecting element (14) attached thereto, which comprises a main body (22) and an electrically insulating plastic, having the following steps:
- Affixing electrical supply leads (26), electrical contacts (24) and antenna (28) in an injection mold,
- 1. First injection procedure, in which the electrical contacts (24), the electrical supply leads (26) and the antenna (28) are partially encased by way of injection molding or casting,
- Affixing the injection-molded part in the injection mold,
- 2. Second injection procedure, in which the electrical supply leads (26), the electrical contacts (24) and the antenna (28) are cast around entirely in order to obtain the final shape of the connecting element.

18. The method as recited in claim 17, **characterized by** the following upstream step:
- Joining the electrical contacts (24) to the electrical supply leads (26), preferably by way of welding.

19. The method according to claim 17, **characterized in that** the electrical supply leads (26), the electrical contacts (24) and the antenna (28) are affixed in the injection mold using retaining means (48).

20. The method according to claim 19, **characterized by** the following downstream steps:
- Removing the retaining means (48), and
- a further injection procedure to fill the cavities (46) created by removing the retaining means (48).

21. The method according to claim 17, **characterized by** the following further steps:
- Pre-assembling of one or more of the electrical contacts (24.1, 24.2) using one or more attachment screws (32.1, 32.2),
- Injection procedure for injection onto or around the cover (38) and for sealing a screw contact with respect to the outside.

22. The method according to claim 17, **characterized in that** a different plastic is used for the second injection procedure than for the first injection procedure.

## Revendications

1. Boîtier (10) destiné à un implant médical, tel qu'un stimulateur cardiaque, un défibrillateur, un cardioverteur ou autres, dans lequel
- le boîtier comporte un boîtier creux (12) ainsi qu'un corps de raccordement (14) fixé au boîtier creux (12), possédant au moins un raccordement électrique installé dans une cavité du corps de raccordement accessible de l'extérieur, pour le raccordement d'une ligne d'électrodes, et
- le corps de raccordement (14) comprend un corps de base (22) constitué d'une matière plastique électriquement isolante, qui est relié au boîtier creux (12) et porte des lignes d'alimentation électrique (26) ainsi que des contacts électriques (24) soudés sur ces lignes d'alimentation electrique de façon électriquement conductrice, en vue du raccordement électrique, de manière à pouvoir relier les contacts (24) avec des composants électriques à l'intérieur du boîtier creux (12), par le biais des lignes d'alimentation électrique (26), dans lequel les lignes d'alimentation électrique et les contacts électriques soudés ensemble sont coulés dans le corps de base ou enrobés par injection et ainsi fixés dans leur position définitive,
**caractérisé en ce que**
- les lignes d'alimentation électrique (26) et les contacts électriques (24) sont fixés par surmoulage ou enrobage par injection, en deux ou plusieurs étapes.

2. Boîtier selon la revendication 1, **caractérisé en ce que** le corps de base (22) est transparent au moins par endroits et laisse voir le raccordement électrique depuis l'extérieur du corps de raccordement.

3. Boîtier selon l'une des revendications 1 ou 2, **caractérisé en ce que** le corps de base (22) est constitué d'une matière plastique transparente apte à être traitée comme une résine à couler ou un thermoplastique.

4. Boîtier selon la revendication 3, **caractérisé en ce que** le corps de base (22) est constitué d'une matière plastique transparente contenant du polyuréthane et/ou une résine époxyde.

5. Boîtier selon l'une des revendications 1 à 4, **caractérisé en ce que** le corps de raccordement (14) est collé avec le boîtier creux (12) au moyen d'une colle.

6. Boîtier selon l'une des revendications 1 à 5, **caractérisé en ce que** le corps de base comporte au moins une autre cavité (36, 40) initialement ouverte vers l'extérieur, pour l'admission de modules fonctionnels (34).

7. Boîtier selon la revendication 6, **caractérisé en ce que** le module fonctionnel est un marqueur de radiographie (42).

8. Boîtier selon la revendication 6, **caractérisé en ce que** le module fonctionnel est un couvercle (38) avec au moins un bouchon d'étanchéité (34.1, 34.2).

9. Boîtier selon la revendication 8, **caractérisé en ce que** le couvercle (38) est collé fermement avec le corps de base (22) à l'aide d'une colle.

10. Boîtier selon l'une des revendications 1 à 9, **caractérisé en ce que** le raccordement électrique est formé par au moins une fiche femelle (16) et un contact électrique (24) disposé dans la fiche femelle (16), la fiche femelle (16) étant conçue pour l'admission d'une fiche mâle d'une ligne d'électrodes.

11. Boîtier selon l'une des revendications 1 à 10, **caractérisé en ce que** les lignes d'alimentation électrique (26) dans le corps de raccordement (14) sont formées par des lignes plates constituées d'un matériau conducteur, comprenant de préférence :
- des aciers résistants à la corrosion,
- du titane pur,
- un alliage de titane,
- du niobium
- un alliage de niobium, ou
- un alliage à base de cobalt, sans fer.

12. Boîtier selon l'une des revendications 1 à 11, **caractérisé en ce qu'**une ligne formant une antenne (28) ensemble avec les lignes d'alimentation est coulée dans le corps de base (22) ou enrobée par injection ensemble avec celui-ci et ainsi fixée dans sa position définitive.

13. Boîtier selon la revendication 12, **caractérisé en ce que** l'antenne (28) est formée par une ligne plate constituée d'un matériau conducteur, comprenant de préférence :
- des aciers résistants à la corrosion,
- du titane pur,
- un alliage de titane,
- du niobium
- un alliage de niobium, ou
- un alliage à base de cobalt, sans fer.

14. Boîtier selon l'une des revendications 11 à 13, **caractérisé en ce que** les lignes d'alimentation électrique (26) et/ou l'antenne (28) formée par des lignes plates constituées d'un matériau conducteur se trouvent dans un plan et sont de préférence fabriquées selon l'utilisation.

15. Boîtier selon l'une des revendications 1 à 14, **caractérisé en ce que** du côté tourné vers le boîtier creux (12), le corps de base (22) comporte deux cavités (20) pour l'admission d'ancres (18) fixées sur le boîtier creux (12), qui, ensemble avec les cavités, assurent un positionnement plus précis du corps de base (22) par rapport au boîtier creux (12) avant et pendant le collage du corps de raccordement (14) avec le boîtier creux (12).

16. Boîtier selon l'une des revendications 1 à 15, **caractérisé en ce que** le boîtier (10) est le boîtier d'un stimulateur cardiaque implantable ou d'un cardioverteur/défibrillateur implantable ou encore d'une combinaison de ceux-ci.

17. Procédé pour la fabrication d'un boîtier destiné à un implant médical, selon l'une des revendications 1 à 16, avec un boîtier creux (12) sur lequel est fixé un corps de raccordement (14) comprenant un corps de base (22) constitué d'une matière plastique électriquement isolante, avec les étapes suivantes :
- la fixation de lignes d'alimentation électrique (26), de contacts électriques (24) et d'une antenne (28) dans un moule d'injection,
- une première étape d'injection, dans laquelle les contacts électriques (24), les lignes d'alimentation électrique (26) et l'antenne (28) sont partiellement enrobés ou surmoulés,
- la fixation de la pièce moulée par injection dans le moule d'injection,
- une deuxième étape d'injection, dans laquelle les lignes d'alimentation électrique (26), les contacts électriques (24) et l'antenne (28) sont entièrement enrobés, pour obtenir la forme définitive du corps de raccordement (14).

18. Procédé selon la revendication 17, **caractérisé par** les étapes suivantes à exécuter préalablement :
- assemblage des contacts électriques (24) avec les lignes d'alimentation électrique (26), de préférence par soudage.

19. Procédé selon la revendication 17, **caractérisé en ce que** les lignes d'alimentation électrique (26), les contacts électriques (24) et l'antenne (28) sont fixés dans le moule d'injection par des éléments de maintien (48).

20. Procédé selon la revendication 19, **caractérisé par** les étapes suivantes, à exécuter par la suite :
- retrait des éléments de maintien (48), et
- nouvelle étape d'injection pour le remplissage des espaces vides (46) apparus lors du retrait des éléments de maintien (48).

21. Procédé selon la revendication 17, **caractérisé par** les étapes supplémentaires suivantes :
- montage préalable d'un ou de plusieurs des contacts électriques (24.1, 24.2) avec une ou plusieurs vis de serrage (32.1, 32.2),
- étape de moulage par injection pour l'enrobage ou le surmoulage du couvercle (38) et l'isolation d'un assemblage par vissage.

22. Procédé selon la revendication 17, **caractérisé en ce que** pour la deuxième étape d'injection, la matière plastique utilisée est différente de celle de la première étape d'injection.
